# EUROPEAN PATENT SPECIFICATION

(11) **EP 3 307 903 B1**
(45) Date of publication and mention of the grant of the patent: **09.08.2023**
(21) Application number: 16738868.5
(22) Date of filing: 10.06.2016
(51) Int. Cl.: A01N 63/32

(54) **PROCESS FOR THE PREPARATION OF KILLER TOXINS**
VERFAHREN ZUR HERSTELLUNG VON KILLER-TOXINEN
PROCÉDÉ DE PRÉPARATION DES KILLER-TOXINES

(30) Priority: 10.06.2015 PL 41263515
(43) Date of publication of application: 18.04.2018
(73) Proprietor: onesano S.A., 41-506 Chorzow (PL)
(72) Inventor: ZAROWSKA, Barbara, 50-319 Wroclaw (PL); POLOMSKA, Xymena, 55-050 Sobótka (PL); GRZEGORCZYK, Monika, 53-650 Wroclaw (PL); REGIEC, Piotr, 55-200 Olawa (PL); CZAPLICKA-PEDZICH, Marta, 59-610 Wlen (PL); SOSNA, Ireneusz, 54-129 Wroclaw (PL); GUDAROWSKA, Ewelina, 55-330 Wilkszyn (PL); NEJMAN, Mariusz, 53-617 Wroclaw (PL); PASLAWSKA, Marta, 50-570 Wroclaw (PL); FIGIEL, Adam, 50-237 Wroclaw (PL); SEROWIK, Malgorzata, 51-165 Wroclaw (PL); BARLÓG, Jerzy, 55-003 Chrzastawa Mala (PL); SZOLTYSIK, Marek, 51-217 Pruszowice (PL)
(74) Representative: Witek, Rafal
(86) International application number: PCT/IB2016/053438
(87) International publication number: WO 2016/199090

(56) References cited:
- Barbara Zarowska: "Biosynteza i charakterystyka toksyn killerowych drozdzy Debaryomyces hansenii. Monografie CXLVI.", , 1 January 2012 (2012-01-01), pages 1-105, XP055295374, Retrieved from the Internet: URL:http://www.dbc.wroc.pl/Content/18755/8 1_Biosynteza i charakterystyka_Zarowska_B.pdf [retrieved on 2016-08-12]
- BARBARA ZAROWSKA, MARIA WOJTATOWICZ, XYMENA POLOMSKA: "Kynetyka procesu biosyntezy toksyn killerowich prez drozdze Debaryomyces hansenii", Inz. Ap. Chem, vol. 48, no. 3 1 January 2009 (2009-01-01), pages 127-129, XP055295373, Retrieved from the Internet: URL:http://inzynieria-aparatura-chemiczna. pl/pdf/2009/2009-3/InzApChem_2009_3_127-12 9.pdf
- MARQUINA D ET AL: "Production and characteristics of Debaryomyces hansenii killer toxin", MICROBIOLOGICAL RESEARCH, FISCHER, JENA, DE, vol. 156, no. 4, 1 January 2001 (2001-01-01), pages 387-391, XP004956564, ISSN: 0944-5013, DOI: 10.1078/0944-5013-00117 cited in the application
- CHRIS PAYNE ET AL: "The Yeast Debaryomyces hansenii as a Short-Term Biological Control Agent against Fungal Spoilage of Sawn Pinus sylvestris Timber", BIOLOGICAL CONTROL, vol. 22, no. 1, 1 September 2001 (2001-09-01), pages 22-28, XP055295328, US ISSN: 1049-9644, DOI: 10.1006/bcon.2001.0953 cited in the application
- RODRIGUEZ-GONZALEZ ROBERT ET AL: "16 strains of Debaryomyces hansenii tested against Penicillium digitatum, a post harvest fungus, affecting citrus crops in Puerto Rico", FASEB JOURNAL, vol. 27, April 2013 (2013-04), page lb123, XP008181182, & JOINT ANNUAL MEETING OF THE ASPET/BPS AT EXPERIMENTAL BIOLOGY (EB); BOSTON, MA, USA; APRIL 20 -24, 2013
- ZAROWSKA BARBARA ET AL: "Selection of preservation conditions for Debaryomyces hansenii yeasts killer toxins", NEW BIOTECHNOLOGY, vol. 31, no. Suppl. S, July 2014 (2014-07) , page S218, XP002760780, & 16TH EUROPEAN CONGRESS ON BIOTECHNOLOGY; EDINBURGH, UK; JULY 13 -16, 2014 cited in the application
- BREUER U ET AL: "Debaryomyces hansenii - an extremophilic yeast with biotechnological potential.", YEAST 2006 UFZ - CENT. OF ENVIRONMENTAL RES. LEIPZIG-HALLE, DEP. OF ENVIRONMENTAL MICROBIOL., PERMOSERSTRASSE 15, D-04318 LEIPZIG, GERMANY. E-MAIL UTA.BREUER@UFZ.DE, vol. 23, no. 6, 2006, pages 415-437, XP002760784,
- Pathissery J Sarlin ET AL: "A MOLASSES BASED FERMENTATION MEDIUM FOR MARINE YEAST BIOMASS PRODUCTION", International Journal of Research in Marine Sciences, 1 January 2013 (2013-01-01), XP055295366, Retrieved from the Internet: URL:http://urpjournals.com/tocjnls/37_13v2 i2_2.pdf

## Description

The object of the invention is a method of obtaining a preparation containing killer toxins, involving yeasts of the *Debaryomyces hansenii* species (also known as *Candida famata*), the preparation being useful in protecting fruit plants, crops and domesticated plants against development of phytopathogenic fungi.

Antagonistic effect of yeast cells, including killer yeasts, against fungi being plant pathogens, is known [Walker et al. 1995, McGuire 1994, Wisniewski et al. 1991].

Antagonistic effect is known for yeast cells from species other than *Debaryomyces hansenii* including killer toxins produced by some of them, against fruit infecting fungi. Protection of apples and pears against *Botrytis cinerea* and *Penicillium expansum* [Nunes et al. 2002, Vinas et al. 1998, Santos et al., 2004], of grapefruits and oranges against *Penicillium digitatum* [Droby et al. 1989, McGuire R.G. 1994 Platania et al., 2012] and of grapes against *Botrytis cinerea* [Santos and Marquina, 2004] has been demonstrated.

Activity of yeast species other than *Debaryomyces hansenii* against *Botrytis cinerea* on grapevine shrubs is also known [Masih et al. 2001].

Antagonistic effect of *Debaryomyces hansenii* yeast species against *Penicillium digitatum* fungi infecting grapefruits and oranges is known [Droby et al. 1989, Arras 1996].

A method of producing killer toxins with various yeast species is known, in a medium containing glucose (10 g/l), peptone proteose (5 g/l) and yeast extract (3 g/l), malt extract (3 g/l) buffered with 0.2 M phosphate-citrate buffer, pH 4.0 [Santos et al., 2004, Marquina et al. 2001].

A method of producing killer toxins with various yeast species is known, including ones belonging to the *Debaryomyces hansenii* species, in a medium containing glucose (20 g/l), bacteriological peptone (20 g/l) and yeast extract (10 g/l), prepared in a phosphate-citrate buffer, pH 4.5-4.6 [Da Silva et al., 2008; Schmitt and Radler, 1987; Wang et al., 2007a, b, Żarowska et al. 2009]. However, the preparations obtained with this method show a relatively low activity. Barbara Zarowska: "Biosynteza i charakterystyka toksyn killerowych drozdzy Debaryomyces hansenii. Monografie CXLVI.",, 1 January 2012 (2012-01-01), pages 1-105 describes culturing Debaryomyces hansenii in complex media for killer toxin production.

Concentration of killer toxin preparations from *Debaryomyces hansenii* yeasts by ultrafiltrating on a membrane with 18 kDa cut-off and evaporating under vacuum is known [2arowska et al., 2014].

Preservation of killer toxin preparations from *Debaryomyces hansenii* yeasts by freeze drying and spray-drying (in laboratory conditions) with dryer inlet temperature of 100°C is known, while the described method does not provide sufficient efficiency of obtaining the preparation in a powder form nor the desired activity level of the preparation [Zarowska et al., 2014].

The object of the invention is to provide a preparation characterized by high biological activity and suitable for protecting fruit plants, crops and domesticated plants against development of phytopathogenic fungi. A further object of the invention is to provide a method of obtaining such preparation.

The object of the invention is a method of obtaining a preparation containing killer toxins, characterized by the fact that the *Debaryomyces hansenii* yeasts, deposited in the CBS under number CBS 140023 are cultured in an aqueous solution, with pH of 3 to 5, containing 2 to 6% weight per volume of beet molasses and 0.5 to 3% weight per volume of corn steep liquor, preferably in temperature of 10 to 20°C, until the *D. hansenii* yeasts reach stationary growth phase, followed by biomass separation, preferably by centrifugation, and concentration of the remaining supernatant by nanofiltration, and with the thus obtained concentrated fluid being optionally preserved for storage purposes, particularly by spray-drying. Also preferably, the concentrated fluid is preserved for storage purposes by freezing in temperature in the range of -15 to -22°C.

In an alternative preferred embodiment, the biomass obtained after centrifugation process and then frozen in temperature of -15 to -22°C is suspended in tap water. Equally preferably, the biomass obtained after centrifugation process is dried by spray-drying.

A further object of the invention is an antifungal preparation, in particular for protection of fruit plants, crops and domesticated plants, characterized by the fact that it comprises killer toxins produced by *Debaryomyces hansenii* yeasts deposited in the CBS under number CBS 140023, wherein activity of the preparation is at least 750 U/ml. Preferably, the preparation according to the invention can be obtained by the above described method from a concentrated post-culture fluid.

A further object of the invention is an antifungal preparation, in particular for protection of fruit plants, crops and domesticated plants, obtained by the above defined method of the invention.

Unexpectedly, it has been found that it is possible to utilize preparations comprising *Debaryomyces hansenii* yeasts killer toxins for protection of fruit plants, crops and domesticated plants against development of phytopathogenic fungi.

Unexpectedly, the present invention provides the method of production of the final preparation containing killer toxins from *Debaryomyces hansenii* yeasts using byproducts from food industry such as beet molasses and corn steep liquor, concentrated by nanofiltration and preserved be freezing or spray-drying with inlet temperature of 140 - 200°C, allowing to obtain preparations with high biological activity.

Preferably, for stabilization of pH in the culture media an inorganic acid is used.

Preferably, the raw material feeding rate into the drying chamber enables reaching an outlet temperature of 70 to 90°C.

Preferably, the spraying conditions enables obtaining droplets with a size of 12 µm to 17 pm.

Preferably, air stream flowing through the chamber enables a proper moisture removing process and allows to obtain a powdered preparation with water activity in the range of 0.142 - 0.165.

An advantage of the invention is the possibility of producing a preparation containing killer toxins from *Debaryomyces hansenii,* having an inhibitory activity against phytopathogenic fungi, using byproducts originating from food industry: beet molasses and corn steep liquor. Such a solution enables production of the preparation being incomparably cheaper than with the known media. Additionally, 15 to 25 g/L of yeast dry mass is obtained, which can also be utilized against phytopathogenic fungi.

The object of the invention is illustrated in the examples.

### Example 1

A *Debaryomyces hansenii* AII4b yeast inoculum is introduced to production media containing 1 L of tap water, 20 g of molasses and 10 g of corn steep liquor, preferably in the amount of 10% of the working volume.

The *Debaryomyces hansenii* AII4b strain was deposited in the 19^{th} May 2015 according to the Budapest Treaty in the Centraalbureau voor Schimmelcultures (CBS) in Utrecht, Netherlands, under the deposition number CBS 140023.

The inoculum is prepared in the same medium, on a shaker with the rate of 150-200 rpm, in temperature of 22°C for 3 days. The medium pH is adjusted to 4.0 and controlled with a HCl solution. The proper culture is conducted in temperature of 14°C, with stirrer rotational speed of 400 rpm and aeration rate of 200 - 600 mL/min until the yeasts reach stationary growth phase. After culture, the biomass is separated from the post-culture fluid by centrifugation. The supernatant, comprising killer toxins is concentrated seven-fold by nanofiltration, followed by drying in a countercurrent spray drier, in the following conditions: raw material temperature of 8°C, inlet temperature of 165°C, raw material feed flow rate of 4750 mL/h, air pressure at the nozzle of 0.27 MPa.

A preparation is obtained having 800 - 900 U/mL water activity with yeast biomass in the amount of 15 g/L.

Killer activity is determined by a diffusion plate assay. To this end, an agar YPG medium, pH 4.6 containing 0.03% methylene blue is inoculated with a sensitive strain culture up to a final density of 5×10⁵ cells/mL. In such prepared plates wells having a 6mm diameter are excised using a cork borer, wherein 100 µL of the preparation comprising killer toxins is introduced. The plates are allowed to stay for about 8 hours in temperature of 4°C for the toxin to diffuse into the medium, followed by incubation in temperature of 14°C for 48 hours. The growth inhibition zones formed around the wells are measured using calipers.

An amount of the killer protein inducing a growth inhibition zone for the sensitive strain (with the radius reduced by the radius of the well) of 2 mm is considered a unit of activity.

A linear relationship was observed between the natural logarithm of the toxin preparation volume (y) and the size of the inhibition zone (x) of the standard sensitive strain Y. *lipolytica* PII6a. For the toxin obtained using the *D. hansenii* AII4b strain this relationship is as follows:
- AII4b: y = 1.5735ln (x) + 1.8645; correlation coefficient R²=0.9791

The toxin preparation, obtained with the above described method, after being dissolved in water to a working concentration of about 8% and used in a form of sprayings during plant's vegetative period, exhibits activity both on leaves and on fruits, particularly against apple scab (*Venturia inaequalis*) and grey mold (*Botrytis cinerea*) of strawberries and grapevine. The sprayings are applied within the period of primary infections.

### Example 2

The procedure is followed as in example 1, however, the supernatant, concentrated by nanofiltration is frozen at the temperature of -20°C. The preparation is characterized by the same activity as the preparation of example 1.

### Example 3

The procedure is followed as in example 1, however, the production medium contains 60 g/l of beet molasses and 10 g/L of corn steep liquor. The obtained preparation is characterized by activity at a level of 750 - 800 U/mL with yeast biomass of 23 g/L. The preparation is characterized by the same activity as the preparation of example 1.

### Example 4

The procedure is followed as in example 1, however the yeast biomass obtained after centrifugation is frozen in temperature of -20°C, then suspended in tap water up to 20 g/l of cell dry mass content and applied in the form of sprayings during plant vegetative period. The preparation exhibits activity both on leaves and on fruits, particularly against apple scab (*Venturia inaequalis*) and grey mold (*Botrytis cinerea*) of strawberries and grapevine.

### Example 5

The procedure is followed as in example 4, however, the yeast biomass obtained after centrifugation is dried in a countercurrent spray drier, in the following conditions: raw material temperature of 8°C, the inlet temperature of 165°C, raw material feed flow rate of 4750 mL/h, air pressure at the nozzle of 0.27 MPa, and then suspended in tap water up to 20 g/Lof cell dry mass content and applied in the form of sprayings during plant vegetative period. The preparation is characterized by the same activity as the preparation of example 4.

References:
1. Arras G., 1996, Mode of action of an isolate of Candida famata in biological control of Penicillium digitatum in orange fruits, Postharv. Biol. Technol., 8, 191-198
2. Da Silva S., Calado S., Lucas C., Aguiar C., 2008, Unusual properties of the halotolerant yeast Candida nodaensis Killer toxin, CnKT, Microbiol. Res., 163, 243-251
3. Droby S., Chalutz E., Wilson C.L., Wisniewski M.E.. 1989. Characterisation of the biological control activity of Debaryomyces hansenii in the control of Penicillium digitatum on grapefruit. Can. J. Microbiol., 35,794-800.
4. Masih E.I, Slezack-Deschaumes S., Marmaras I., Ait Barka E., Vernet G., Charpentier C., Adholeya A., Paul B. 2001. Characterization of the yeast Pichia membranifaciens and its possible use in the biological control of Botrytis cinerea, causing the grey mould disease of grapevines. FEMSMicrobiol. Lett. 202, 227-232.
5. McGuire R.G. 1994. Application of Candida guillermondii in commercial citrus coatings for biological control of Penicillium digitatum on grapefruits. Biol. Control, 4, 1-7.
6. Nunes C., Usall J., Teixidó N., Fons E., Viñas I., 2002. Postharvest biological control by Pantoea agglomerans (CPA-2) on golden delicious apples. Journal of Applied Microbiology, 92: 247-255.
7. Payne Ch., Bruce A., 2001, The Yeast Debaryomyces hansenii as a short-term biological control agent against fungal spoilage of sawn Pinus sylvestris timber, Biol. Control, 22, 22-28
8. Platania C, Restuccia C, Muccilli S, Cirvilleri G., 2012, Efficacy of killer yeasts in the biological control of Penicillium digitatum on Tarocco orange fruits (Citrus sinensis), Food Microbiol., 30 (1), 219-25
9. Santos A. Sánchez A., Marqiuna D., 2004, Yeasts as biological agents to control Botrytis cinerea, Microbiol. Res., 159, 331-338
10. Santos A., Marquina D., 2004, Killer toxin of Pichia membranifaciens and its possible use as a biocontrol agent against grey mould disease of grapevine, Microbiol., 150, 2527-2534
11. Schmitt M.J., Radler F., 1987, Mannoprotein of the yeast cell wall as primary receptor for the killer toxin of Saccharomyces cerevisiae strain 28, J. Gen. Microbiol., 28, 3346-3354
12. Vinas, I., Usall, J., Teixido', N., Sanchis, V., 1998. Biological control of major postharvest pathogens on apple with Candida sake. Int. J. Food Microbiol. 40, 9-16.
13. Walker G.M., McLeod A.H., Hodgson V.J. 1995. Interactions between killer yeasts and pathogenic fungi. FEMS Microbiol. Lett. 127, 213-222.
14. Wang X., Chi Z., Yue L., Li J., Li M., Wu L., 2007a, A marine killer yeast against the pathogenic yeast strain in crab (Portunus trituberculatus) and an optimization of the toxin production, Microbiol Res, 162(1), 77-85
15. Wang X., Chi Z., Yue L., Li J., Li M., Wu L., 2007b, Purifiction and characterization of killer toxin from a marine killer yeast Pichia anomala YF07b against the pathogenic yeast strain in crab, Curr. Microbiol., 55, 396-401
16. Wisniewski M.E., Biles C.L., Droby S., McLaughlin R.J., Wilson C.L., Chalutz E. 1991. Mode of action of the post harvest biocontrol yeast Pichia guillermondii. Characterization of attachment to Botrytis cinerea. Physiol. Mol. Plant Pathol. 39, 245-248.
17. Żarowska B., Bobak L, Regiec P., Figiel A., Grzegorczyk M., Szoltysik M., Wojtatowicz M., Polomska X., 2014, Selection of preservation conditions for Debaryomyces hansenii yeasts killer toxins,, New Biotechnology, 31, 218
18. Żarowska B., Wojtatowicz M., Polomska X. 2009. Kinetyka procesu biosyntezy toksyn killerowych przez drozdze Debaryomyces hansenii. Inzynieria i aparatura chemiczna, 3, 127-129.

## Claims

1. A method of obtaining a preparation comprising killer toxins, **characterized in that** the *Debaryomyces hansenii* yeasts, deposited in the CBS under number CBS 140023 are cultured in an aqueous solution, with pH of 3 to 5, containing 2 to 6% weight per volume of beet molasses and 0.5 to 3% weight per volume of corn steep liquor, preferably in temperature of 10 to 20°C, until the *D. hansenii* yeasts reach stationary growth phase, followed by biomass separation, preferably by centrifugation, and concentration of the remaining supernatant by nanofiltration, and with the thus obtained concentrated fluid being preserved for storage purposes, particularly by spray-drying.

2. The method according to claim 1, **characterized in that** the concentrated fluid is preserved for storage purposes by freezing in temperature in the range of -15 to -22°C.

3. The method according to claim 1, **characterized in that** the biomass obtained after centrifugation process and then frozen in temperature of -15 to -22°C is suspended in tap water.

4. The method according to claim 3, **characterized in that** the biomass obtained after centrifugation process is dried by spray-drying.

5. An antifungal preparation, in particular for protection of fruit plants, crops and domesticated plants, **characterized in that** it comprises killer toxins produced by *Debaryomyces hansenii* yeasts deposited in the CBS under number CBS 140023, wherein activity of the preparation is at least 750 U/mL.

6. The preparation according to claim 5, **characterized in that** it was obtained by the method defined in claim 1 to 2.

7. An antifungal preparation, in particular for protection of fruit plants, crops and domesticated plants, obtained by the method defined in claim 1-4.

## Patentansprüche

1. Verfahren zum Gewinnen eines Präparats, das Killertoxine enthält, **dadurch gekennzeichnet, dass** die *Debaryomyces-hansenii*-Hefen, die beim CBS (Centraalbureau voor Schimmelcultures) unter der Nummer CBS 140023 deponiert sind, in einer wässrigen Lösung mit einem pH-Wert von 3 bis 5, die 2 bis 6 % Gewicht pro Volumen Rübenmelasse und 0,5 bis 3 % Gewicht pro Volumen Corn Steep Liquor enthält, vorzugsweise bei einer Temperatur von 10 bis 20 °C kultiviert werden, bis die D.-hansenii-Hefen die stationäre Wachstumsphase erreichen, gefolgt von Separation von Biomasse, vorzugsweise durch Zentrifugieren sowie Konzentration des verbleibenden Überstandes durch Nanofiltration, und wobei die so gewonnene konzentrierte Flüssigkeit, insbesondere mittels Sprühtrocknung, für Lagerungs-zwecke konserviert wird.

2. Verfahren nach Anspruch 1, **dadurch gekennzeichnet, dass** die konzentrierte Flüssigkeit mittels Einfrierens bei einer Temperatur im Bereich von -15 bis -22 °C für Lagerungszwecke konserviert wird.

3. Verfahren nach Anspruch 1, **dadurch gekennzeichnet, dass** die nach dem Zentrifugiervorgang gewonnene und dann bei einer Temperatur von -15 bis -22°C eingefrorene Biomasse in Leitungswasser suspendiert wird.

4. Verfahren nach Anspruch 3, **dadurch gekennzeichnet, dass** die nach dem Zentrifugiervorgang gewonnene Biomasse mittels Sprühtrocknung getrocknet wird.

5. Fungizides Präparat, insbesondere zum Schutz von Obstgewächsen, Nutzpflanzen und Kulturpflanzen, **dadurch gekennzeichnet, dass** es Killertoxine enthält, die von *Debaryomyces-*hansenii-Hefen produziert werden, die beim CBS unter der Nummer CBS 140023 deponiert sind, wobei die Aktivität des Präparats wenigstens 750 U/mL beträgt.

6. Präparat nach Anspruch 5, **dadurch gekennzeichnet, dass** es mit dem in den Ansprüchen 1 bis 2 definierten Verfahren gewonnen wurde.

7. Fungizides Präparat, insbesondere zum Schutz von Obstgewächsen, Nutzpflanzen und Kulturpflanzen, gewonnen mit dem in den Ansprüchen 1 - 4 definierten Verfahren.

## Revendications

1. Méthode d'obtention d'une préparation comprenant des toxines tueuses, **caractérisée en ce que** les levures *Debaryomyces hansenii,* déposées auprès du CBS sous le numéro CBS 140023 sont cultivées dans une solution aqueuse, avec un pH de 3 à 5, contenant de 2 à 6 % en poids par volume de mélasse de betterave et de 0,5 à 3 % en poids par volume d'extrait soluble de maïs, de préférence à une température de 10 à 20 °C, jusqu'à ce que les levures de *D*. *hansenii* atteignent une phase de croissance stationnaire, suivie par une séparation de biomasse, de préférence par centrifugation, et une concentration du surnageant restant par nanofiltration, et le fluide concentré ainsi obtenu étant conservé à des fins de stockage, notamment par séchage par pulvérisation.

2. Méthode selon la revendication 1, **caractérisée en ce que** le fluide concentré est conservé à des fins de stockage par congélation à une température dans la plage de -15 à -22 °C.

3. Méthode selon la revendication 1, **caractérisée en ce que** la biomasse obtenue après un procédé de centrifugation puis congelée à une température de -15 à -22 °C est mise en suspension dans de l'eau du robinet.

4. Méthode selon la revendication 3, **caractérisée en ce que** la biomasse obtenue après le procédé de centrifugation est séchée par séchage par pulvérisation.

5. Préparation antifongique, en particulier pour la protection d'arbres fruitiers, de cultures et de plantes domestiquées, **caractérisée en ce qu'**elle comprend des toxines tueuses produites par des levures *Debaryomyces hansenii* déposées auprès du CBS sous le numéro CBS 140023, dans laquelle l'activité de la préparation est d'au moins 750 U/ml.

6. Préparation selon la revendication 5, **caractérisée en ce qu'**elle a été obtenue par la méthode définie dans les revendications 1 à 2.

7. Préparation antifongique, en particulier pour la protection d'arbres fruitiers, de cultures et de plantes domestiquées, obtenue par la méthode définie dans les revendications 1 à 4.
